# EUROPEAN PATENT SPECIFICATION

(11) **EP 4 604 900 B1**
(45) Date of publication and mention of the grant of the patent: **03.12.2025**
(21) Application number: 23818249.7
(22) Date of filing: 25.10.2023
(51) Int. Cl.: A61G 13/02, A61B 6/10, A61G 13/10

(54) **BRAKING MECHANISM AND MEDICAL TABLE**
BREMSMECHANISMUS UND MEDIZINISCHER TISCH
MÉCANISME DE FREINAGE ET TABLE MÉDICALE

(30) Priority: 23.11.2022 CN 202223120399 U
(43) Date of publication of application: 27.08.2025
(73) Proprietor: Siemens Shanghai Medical Equipment Ltd., 201318 Shanghai (CN)
(72) Inventor: WANG, Ji Zhao, Shanghai 201204 (CN)
(74) Representative: HKW Intellectual Property PartG mbB
(86) International application number: PCT/CN2023/126563
(87) International publication number: WO 2024/109429

(56) References cited:
- EP-B1- 3 431 321
- US-A1- 2005 129 181
- US-A1- 2014 192 956
- US-A1- 2021 059 883

## Description

### TECHNICAL FIELD

The present application relates to a braking mechanism and a medical table including the braking mechanism.

### BACKGROUND

In the medical field, the table board of the medical table is typically arranged to be able to move relative to the table frame, so as to facilitate examination and treatment. An electromagnetic braking mechanism is often used for braking between the table board and the table frame. In the existing electromagnetic braking mechanism, when the electromagnet is energized, due to the effect of the magnetic force, the electromagnet is attracted by the table board and quickly moves until it abuts against the table board. Since the electromagnet moves at a very high speed, the collision noise when the electromagnet contacts the table board is very loud.

EP 3431321 B1 relates to a braking device for a moving body, such as a railroad vehicle or the like, which performs braking by using an eddy- current force between an electromagnet placed on the movable object and a rail. At the time of starting operation as an emergency brake in an emergency, coils are energized so that an electromagnetic force (attraction force) acts between the rail and the brake electromagnet. Thus, the brake electromagnet moves against impelling by springs toward the rail, so that the rail and the brake pad make contact with each other. On this occasion, a braking force due to frictional force between the rail and the brake pad is added to a first-stage braking force due to eddy current.

US 2014/192956 A1 discloses an X-ray imaging apparatus having an improved structure to reduce noise generated when a position of an X-ray generator is fixed in position includes an X-ray generator, an X-ray detector, a moving carriage coupled with one end of the X-ray generator, a guide rail, and a holding unit disposed at an inner side of the moving carriage to hold the position of the X-ray generator, wherein the holding unit includes a contact pad to contact the guide rail to hold the moving carriage, a magnetic body to move the contact pad using magnetic force, and a holding plate coupled to the contact pad to move according to operation of the magnetic body and including a buffer material capable of absorbing noise or vibration.

US 2021/059883 A1 discloses that a medical suspension bridge includes a cross beam, a suspension pipe and a moving module. The cross beam is connected with the suspension pipe. The moving module is movably connected with the cross beam. The cross beam includes a load-bearing beam. The moving module includes a cable carrier moving plate extending to the upper portion of the load-bearing beam. The medical suspension bridge further includes a cable carrier. One end of the cable carrier is connected to the load-bearing beam, and the other end is connected to the cable carrier moving plate. Since cables such as electric wires and air pipes within a box body which needs to be moved are collectively mounted within the cable carrier, not only the routing is more tidy, but also the cables can be well protected.

US 2005/129181 A1 discloses that a diagnostic table for a medical imaging apparatus includes a sliding command input device by which a sliding command is configured to be input to produce a sliding movement of a tabletop, a first detector configured to detect sliding movement of the tabletop, a second detector configured to detect input of the sliding command, a stopper coupled to the tabletop and configured to prevent the tabletop from sliding, and a controller coupled to the first and second detectors and the stopper, the controller configured to determine a fault condition when the first detector detects sliding movement of the tabletop inconsistent with the command detected by the second detector and to activate the stopper to prevent the sliding movement of the tabletop upon determining existence of the fault condition.

### SUMMARY

An objective of the present application is to provide a braking mechanism, which is conducive to reducing noise generated during braking.

Another objective of the present application is to provide a medical table, whose braking mechanism is conducive to reducing noise generated during braking.

The invention is defined by the attached set of claims. Further details of the disclosed method, devices and system are described below, which are helpful for understanding the claimed invention.

The present application provides a braking mechanism, which includes a first support, a first magnet and a driving unit. The first support is configured to connect a first object. The first magnet is movably connected to the first support along a first straight line. The first magnet is selected from an electromagnet and an electrically-controlled permanent magnet. The driving unit is capable of driving the first magnet to move along the first straight line relative to the first support to enable the first magnet to reciprocate between a release position and a braking position. The first magnet in the braking position is configured to contact and magnetically attract a second object movable relative to the first object.

During braking of the braking mechanism, the driving unit firstly drives the first magnet to the braking position to contact the second object, and then controls the first magnet to generate a magnetic field to attract the second object. By properly arranging the driving unit, the noise generated when the first magnet comes into contact with the second object can be reduced to an acceptable range. The arrangement of the driving unit does not affect the magnitude of the magnetic attraction of the first magnet to the second object. Thereby, on the basis of ensuring the braking effect, the braking mechanism is conducive to effectively reducing the noise generated during braking.

**In** another schematic embodiment of the braking mechanism, the driving unit includes a first elastic member and a driving assembly. The first elastic member is capable of applying an elastic force to drive the first magnet to move between the release position and the braking position along a first direction parallel to the first straight line relative to the first support. The driving assembly includes an iron core and a second magnet. The iron core movably runs through the second magnet along the first straight line. The second magnet is selected from an electromagnet and an electrically-controlled permanent magnet. A magnetic field generated by the second magnet is capable of driving the iron core to move along the first straight line relative to the second magnet. One of the iron core and the second magnet is fixedly arranged relative to the first support. The other one of the iron core and the second magnet is fixedly arranged relative to the first magnet to drive the iron core to move by the second magnet to drive the first magnet to move between the release position and the braking position along a direction opposite to the first direction. This structure is simple and low in cost.

In still another schematic embodiment of the braking mechanism, the braking mechanism further includes a second support. The second support is movably connected to the first support along the first straight line. The first magnet and the second magnet are fixedly arranged on the second support. The iron core is fixedly arranged on the first support. In this way, the assembly is facilitated.

In yet another schematic embodiment of the braking mechanism, the first support has a first flat plate portion. The second support has a second flat plate portion. The first flat plate portion and the second flat plate portion are both arranged perpendicular to the first direction and overlapped with each other along the first direction. The first magnet and the second magnet are arranged on a side of the second flat plate portion facing away from the first flat plate portion. In this way, the structure is more compact and the space can be saved.

In yet another schematic embodiment of the braking mechanism, one of the first flat plate portion and the second flat plate portion is provided with a slide hole penetrating along the first direction, and the other one of the first flat plate portion and the second flat plate portion is provided with a slide bar. The slide bar slidably runs through the slide hole along the first direction. In this way, the structure is simple and stable.

In yet another schematic embodiment of the braking mechanism, the first direction is a direction in which the first magnet moves from the braking position to the release position. The second flat plate portion has the slide hole. The first support has the slide bar. An end of the slide bar located on the second flat plate portion and facing away from the first flat plate portion has a boss portion. The first elastic member is a compression spring and is sleeved on the slide bar. One end of the first elastic member abuts against the second flat plate portion, the first magnet or the second magnet, and the other end abuts against the boss portion. In this way, the structure is simple and stable.

In yet another schematic embodiment of the braking mechanism, the first direction is a direction in which the first magnet moves from the release position to the braking position. The second flat plate portion has an avoiding hole. The first magnet has a first accommodating cavity opposite to the avoiding hole along the first direction. The first elastic member runs through the avoiding hole. One end of the first elastic member abuts against the first flat plate portion, and the other end extends into the first accommodating cavity and abuts against the first magnet. The structure is simple and the space can be saved.

In yet another schematic embodiment of the braking mechanism, the braking mechanism is provided with a plurality of first magnets. At least a part of the plurality of first magnets are electrically-controlled permanent magnets. The advantage of arranging the electrically-controlled permanent magnets is that the electrically-controlled permanent magnets can maintain their magnetism after an accidental power failure of the system, and thus, can be used for avoiding a braking failure after the power failure.

In yet another schematic embodiment of the braking mechanism, the braking mechanism further includes a connecting member, a second elastic member and an abutting member. The connecting member is movably connected to the first support along the first straight line. The first support is connected to the first object through the connecting member. The second elastic member is capable of applying an elastic force to drive the first support to move relative to the connecting member in a direction the same as the direction in which the first magnet moves from the release position to the braking position. The abutting member is connected to the first support to abut against the second object under the action of the elastic force of the second elastic member. When in use, even if a relative distance between the first object and the second object along the first straight line is slightly offset, the abutting member can also abut against the second object under the action of the elastic force of the second elastic member, so as to keep the relative position between the first support and the second object along the first straight line constant, thereby ensuring that the first magnet in the braking position can contact the second object.

In yet another schematic embodiment of the braking mechanism, the abutting member is a roller wheel, and the roller wheel is rotatably connected to the first support with a rotation axis perpendicular to the first straight line. In this way, mutual friction between the abutting member and the second object can be reduced.

In yet another schematic embodiment of the braking mechanism, the connecting member is a pin and runs through the first support. The second elastic member is a compression spring and is sleeved on the connecting member. This structure is simple and low in cost.

In yet another schematic embodiment of the braking mechanism, the braking mechanism is provided with a plurality of connecting members. The first support includes a first flat plate portion and a plurality of connecting portions. The first flat plate portion is in a flat plate shape perpendicular to the first straight line. Each of the connecting portions is in a flat plate shape parallel to the first flat plate portion. Each of the connecting members runs through one of the connecting portions. The first magnet, the abutting member and the connecting portions are located on a same side of the first flat plate portion. In this way, the structure is more compact and the space can be saved.

The present application provides a medical table, which includes a table frame, a table board and a braking mechanism described above. The table board is movably arranged on the table frame. The first support is connected to one of the table frame and the table board. The other one of the table frame and the table board contacts the first magnet in the braking position and is capable of being magnetically attracted by the magnetic force of the first magnet. On the basis of ensuring the braking effect, the braking mechanism of the medical table is conducive to effectively reducing the noise generated during braking.

### BRIEF DESCRIPTION OF THE DRAWINGS

The following accompanying drawings merely illustrate and explain the present application schematically, and do not limit the scope of the present application.
FIG. 1 is a three-dimensional view of a schematic embodiment of a braking mechanism.
FIG. 2 is a partial sectional view of the braking mechanism shown in FIG. 1.
FIG. 3 is a schematic exploded view of the structure shown in FIG. 2.
FIG. 4 is a schematic view of another state of the structure shown in FIG. 2.
FIG. 5 is a partial sectional view of another schematic embodiment of a braking mechanism.
FIG. 6 is a partial sectional view of still another schematic embodiment of a braking mechanism.
FIG. 7 is a schematic exploded view of the structure shown in FIG. 6.
FIG. 8 is a schematic view of another state of the structure shown in FIG. 6.
FIG. 9 is a schematic structural view of a schematic embodiment of a medical table.

### Description of symbols

- 100: braking mechanism
- 10: first support
- 11: first flat plate portion
- 12: connecting portion
- 20: second support
- 21: second flat plate portion
- 211: avoiding hole
- 30: first magnet
- 31: first accommodating cavity
- 41: slide hole
- 42: slide bar
- 421: boss portion
- 51: first elastic member
- 52: driving assembly
- 521: iron core
- 522: second magnet
- 71: connecting member
- 72: second elastic member
- 73: abutting member
- 81: table frame
- 82: table board
- 91: first object
- 92: second object
- L1: first straight line
- D1: first direction

### DETAILED DESCRIPTION

For a clearer understanding of the technical features, objectives and effects of the present application, the specific embodiments of the present application will be described with reference to the accompanying drawings, in which like reference numerals refer to components having the same structure or similar structure but having the same function.

Here, "schematic" means "serving as an example, instance or illustration", and any illustration or embodiments described herein as "schematic" should not be interpreted as a more preferred or more advantageous technical solution.

Herein, "first", "second" and the like are not intended to indicate their importance or sequence and the like, but only used for distinguishing each other to facilitate the description of the document.

For the sake of brevity, only the parts related to the present application are schematically shown in the drawings, but they do not represent the actual structure of the product.

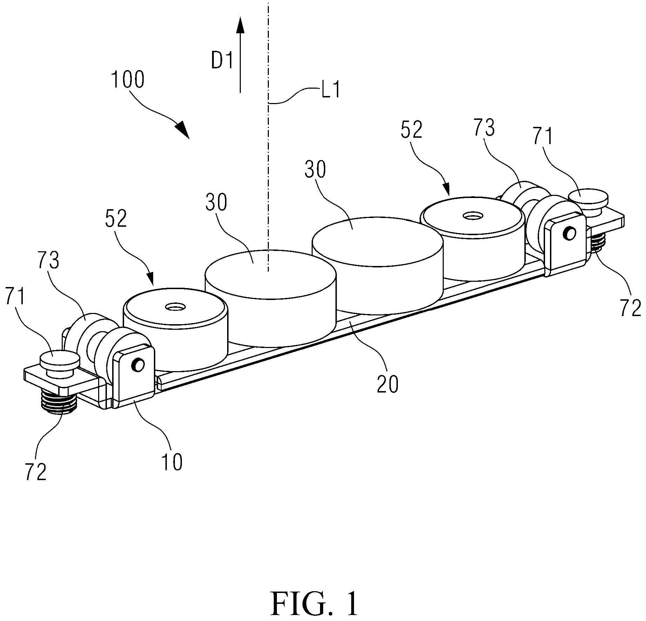

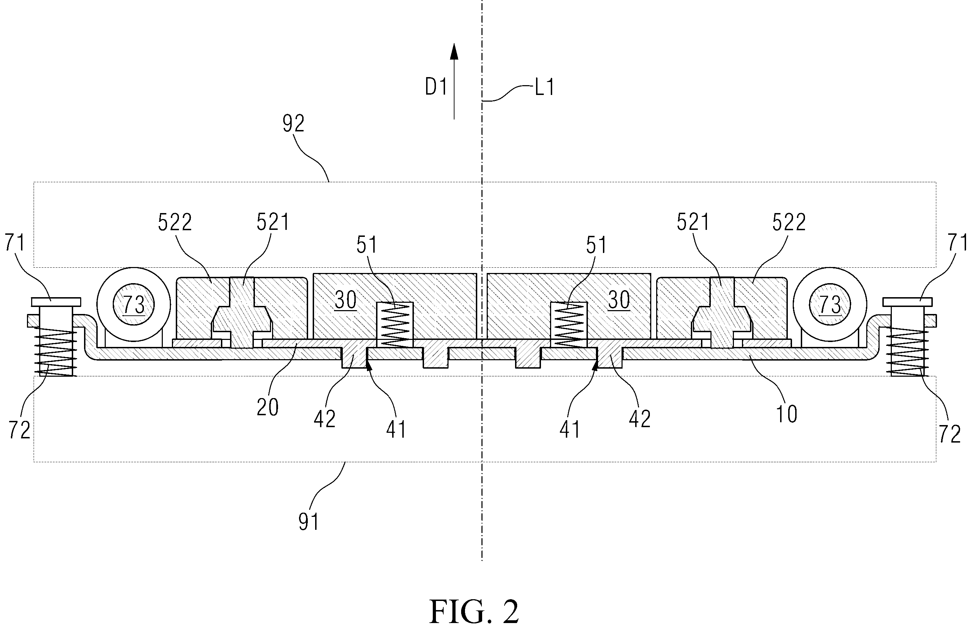

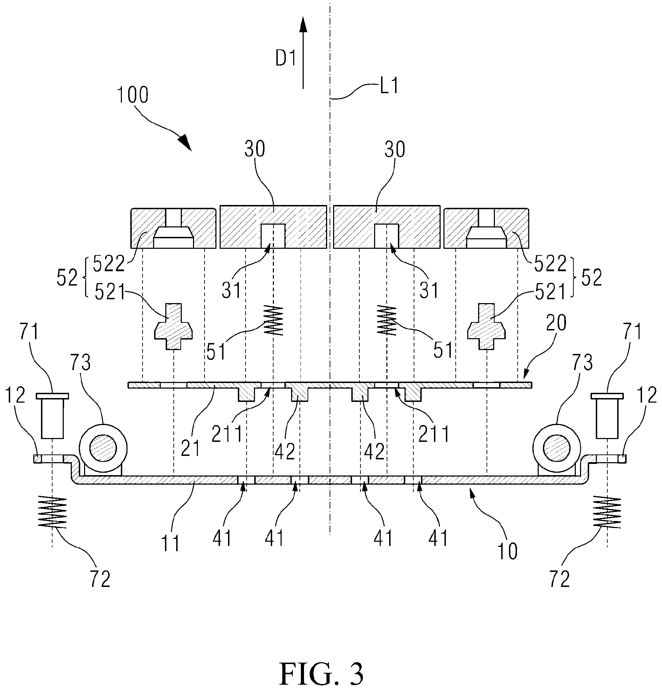

FIG. 1 is a three-dimensional view of a schematic embodiment of a braking mechanism. FIG. 2 is a partial sectional view of the braking mechanism shown in FIG. 1. FIG. 3 is a schematic exploded view of the structure shown in FIG. 2. The braking mechanism is, for example, used for braking a first object 91 and a second object 92 that are movable relative to each other in a direction, for example, perpendicular to a first straight line L1 in the figure. For example, the braking mechanism may be a braking mechanism for a table structure. In this case, the first object 91 and the second object 92 are, for example, but not limited to, a table frame and a table board movably connected to the table frame respectively. As shown in FIG. 1 to FIG. 3, a braking mechanism 100 includes a first support 10, two first magnets 30 and a driving unit.

Each of the first magnet 30 is movably connected to the first support 10 along the first straight line L1. Specifically, in this schematic embodiment, the braking mechanism 100 further includes a second support 20. The second support 20 is movably connected to the first support 10 along the first straight line L1. The first magnets 30 are fixedly arranged on the second support 20, that is, the first magnets 30 are movably connected to the first support 10 through the second support 20.

Further, as shown in FIG. 3, in this schematic embodiment, the first support 10 has a first flat plate portion 11. The second support 20 has a second flat plate portion 21. The first flat plate portion 11 and the second flat plate portion 21 are both arranged perpendicular to the first straight line L1 and overlapped with each other along a direction parallel to the first straight line L1. The first magnets 30 are arranged on a side of the second flat plate portion 21 facing away from the first flat plate portion 11. In this way, the overall structure is more compact and the space can be saved.

Further, as shown in FIG. 3, in this schematic embodiment, the first flat plate portion 11 is provided with a plurality of slide holes 41 penetrating along the first straight line L1, and the second flat plate portion 21 is provided with a plurality of slide bars 42 (only two slide bars 42 are shown in FIG. 3). Each of the slide bars 42 slidably runs through one slide hole 41 along the direction parallel to the first straight line L1. In this way, the movable connection between the first support 10 and the second support 20 is realized through the slide hole 41 and the slide bar 42. However, the present application is not limited thereto, in other schematic embodiments, it may also be that the second flat plate portion 21 is provided with the slide hole 41, and the first flat plate portion 11 is provided with the slide bar 42. The structure of the slide hole and the slide bar is simple and stable, but the present application is not limited thereto. In other schematic embodiments, the movable connection between the first support 10 and the second support 20 may also be realized through other structures.

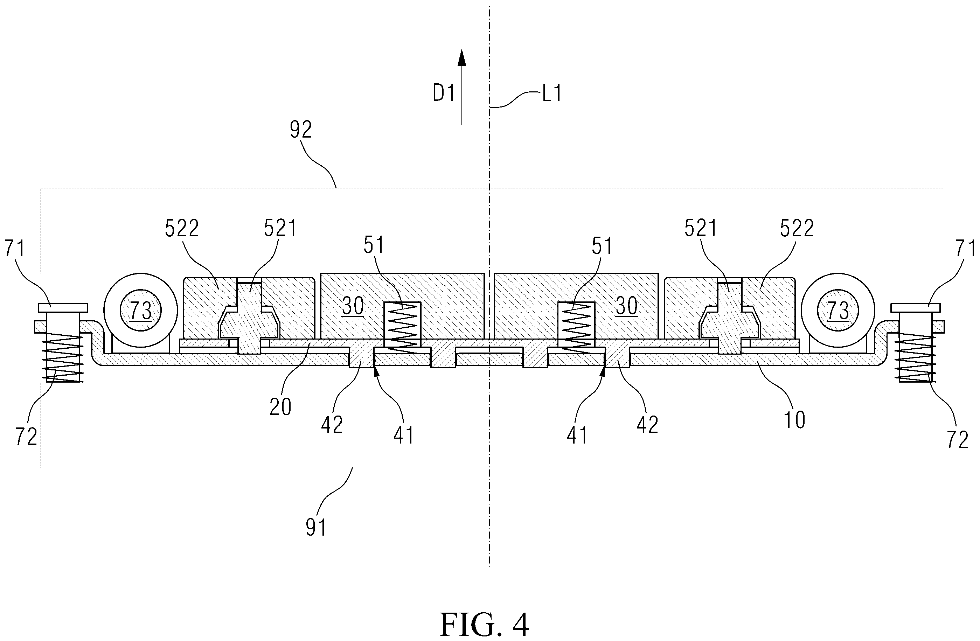

The driving unit is capable of driving the first magnet 30 to move along the first straight line L1 relative to the first support 10 to enable the first magnet 30 to reciprocate between a release position and a braking position. The first magnet 30 shown in FIG. 2 is in the release position. FIG. 4 is a schematic view of another state of the structure shown in FIG. 2, and the first magnet 30 shown in FIG. 4 is in the braking position. The first magnet 30 in the braking position is configured to contact and magnetically attract the second object 92.

Specifically, as shown in FIG. 2 and FIG. 3, in this schematic embodiment, the driving unit includes two first elastic members 51 and two driving assemblies 52. The first elastic member 51 is capable of applying an elastic force to drive the first magnet 30 to move from the release position to the braking position along a first direction D1 parallel to the first straight line L1 relative to the first support 10. As shown in FIG. 3, in this schematic embodiment, the second flat plate portion 21 has two avoiding holes 211. Each of the first magnets 30 has a first accommodating cavity 31 opposite to one of the avoiding holes 211 along the first direction D1. Each of the first elastic members 51 runs through one of the avoiding holes 211. One end of the first elastic member 51 abuts against the first flat plate portion 11, and the other end extends into the first accommodating cavity 31 and abuts against the first magnet 30. In this way, the overall structure is more compact and the space can be saved, but the present application is not limited thereto.

As shown in FIG. 2 and FIG. 3, each driving assembly 52 includes an iron core 521 and a second magnet 522. The iron core 521 movably runs through the second magnet 522 along the first straight line L1. The second magnet 522 is selected from an electromagnet and an electrically-controlled permanent magnet. A magnetic field generated by the second magnet 522 is capable of driving the iron core 521 to move along the first straight line L1 relative to the second magnet 522, based on a principle similar to that of a push-pull electromagnet. The iron core 521 is fixedly arranged on the first support 10. The second magnet 522 is fixedly arranged on the second support 20 to drive the iron core 521 to move by the second magnet 522, so as to overcome the elastic force of the first elastic member 51 and drive the first magnet 30 to move from the braking position to the release position along a direction opposite to the first direction D1. The second magnet 522 is arranged on a side of the second flat plate portion 21 facing away from the first flat plate portion 11 to save the space, but the present application is not limited thereto.

The first support 10 is configured to connect the first object 91. Specifically, as shown in FIG. 2 and FIG. 3, in this schematic embodiment, the braking mechanism 100 further includes two connecting members 71, two second elastic members 72 and two abutting members 73. The connecting member 71 is movably connected to the first support 10 along the first straight line L1. The connecting member 71 is, for example, a pin, and runs through the first support 10, but the present application is not limited thereto. When in use, as shown in FIG. 2, the connecting member 71 is fixedly connected to the first object 91, and thereby, the first support 10 is connected to the first object 91 through the connecting member 71. The second elastic member 72 is capable of applying an elastic force to drive the first support 10 to move relative to the connecting member 71 in a direction the same as the direction in which the first magnet 30 moves from the release position to the braking position. The second elastic member 72 is, for example, a compression spring, and is sleeved on the connecting member 71, but the present application is not limited thereto. The abutting member 73 is connected to the first support 10 to abut against the second object 92 under the action of the elastic force of the second elastic member 72. When in use, even if a relative distance between the first object 91 and the second object 92 along the first straight line L1 is slightly offset, the abutting member 73 can also abut against the second object 92 under the action of the elastic force of the second elastic member 72, so as to keep the relative position between the first support 10 and the second object 92 along the first straight line L1 constant, thereby ensuring that the first magnet 30 in the braking position can contact the second object 92. In other schematic embodiments, if the relative distance between the first object 91 and the second object 92 along the first straight line L1 is accurately controlled, the first support 10 may also be directly fixedly connected to the first object 91, and there is no need to provide the connecting member 71, the second elastic member 72 and the abutting member 73. In this schematic embodiment, the abutting member 73 is, for example, a roller wheel, and the roller wheel is rotatably connected to the first support 10 with a rotation axis perpendicular to the first straight line L1 and the direction in which the first object 91 and the second object 92 move relative to each other, thereby reducing the mutual friction between the abutting member and the second object.

In this schematic embodiment, the first magnet 30 is selected from an electromagnet and an electrically-controlled permanent magnet. That is, the two first magnets 30 are both electromagnets; or the two first magnets 30 are both electrically-controlled permanent magnets; or one of the first magnets 30 is an electromagnet, and the other one of the first magnets 30 is an electrically-controlled permanent magnet. The advantage of arranging the electrically-controlled permanent magnets is that the electrically-controlled permanent magnets can maintain their magnetism after an accidental power failure of the system, and thus, can be used for avoiding a braking failure after the power failure.

A schematic usage scenario of the braking mechanism is described below. Before braking, the driving unit is controlled to keep the first magnet 30 in the release position, and the first magnet 30 is controlled to make its magnetic field disappear. During braking, firstly, the driving unit is controlled to make the first magnet 30 move from the release position to the braking position to contact the second object 92, and then the first magnet 30 is controlled to generate the magnetic field to attract the second object 92. To cancel the braking, firstly, the first magnet 30 is controlled to make its magnetic field disappear, and then the driving unit is controlled to make the first magnet 30 move from the braking position to the release position.

Specifically, in this schematic embodiment, the control of the driving unit is realized by controlling the disappearance and generation of the magnetic field of the second magnet 522. In this schematic embodiment, for the electromagnet, when the electromagnet is energized, the magnetic field is generated; and when the electromagnet is deenergized, the magnetic field disappears. For the electrically-controlled permanent magnet, when the electrically-controlled permanent magnet is energized, the magnetic field disappears; and when the electrically-controlled permanent magnet is deenergized, the magnetic field is generated. In this way, the first magnet 30 and the second magnet 522 are controlled.

During braking of the braking mechanism, the driving unit firstly drives the first magnet 30 to the braking position to contact the second object 92, and then controls the first magnet 30 to generate a magnetic field to attract the second object 92. By properly arranging the driving unit, the noise generated when the first magnet 30 comes into contact with the second object 92 can be reduced to an acceptable range. The arrangement of the driving unit does not affect the magnitude of the magnetic attraction of the first magnet 30 to the second object 92. Thereby, on the basis of ensuring the braking effect, the braking mechanism is conducive to effectively reducing the noise generated during braking.

The driving unit of this schematic embodiment is simple in structure and low in cost, but the present application is not limited thereto. In other schematic embodiments, the driving unit may also be other structures, as long as it can drive the first magnet 30 to reciprocate between the release position and the braking position, for example, the driving unit may be a motor or a push-pull electromagnet.

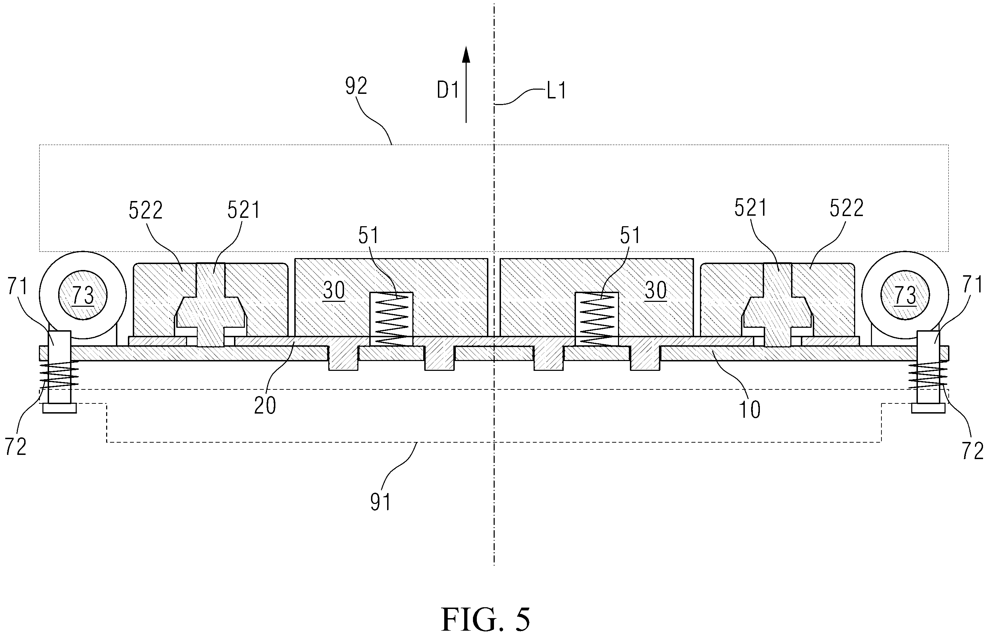

As shown in FIG. 3, in this schematic embodiment, the first support 10 further includes two connecting portions 12. Each of the connecting portions 12 is in a flat plate shape parallel to the first flat plate portion 11. Each of the connecting members 71 runs through one of the connecting portions 12. The second flat plate portion 21, the driving assembly 52, the first magnet 30, the abutting member 73 and the connecting portions 12 are located on the same side of the first flat plate portion 11. In this way, the overall structure is more compact and the space can be saved, but the present application is not limited thereto. In other schematic embodiments, as shown in FIG. 5, the first support 10 may also not be provided with the connecting portions 12, and the connecting member 71 runs through the first flat plate portion 11.

In other schematic embodiments, the numbers of the first magnet 30, the first elastic member 51, the driving assembly 52, the connecting member 71, the second elastic member 72 and the abutting member 73 may be adjusted according to needs.

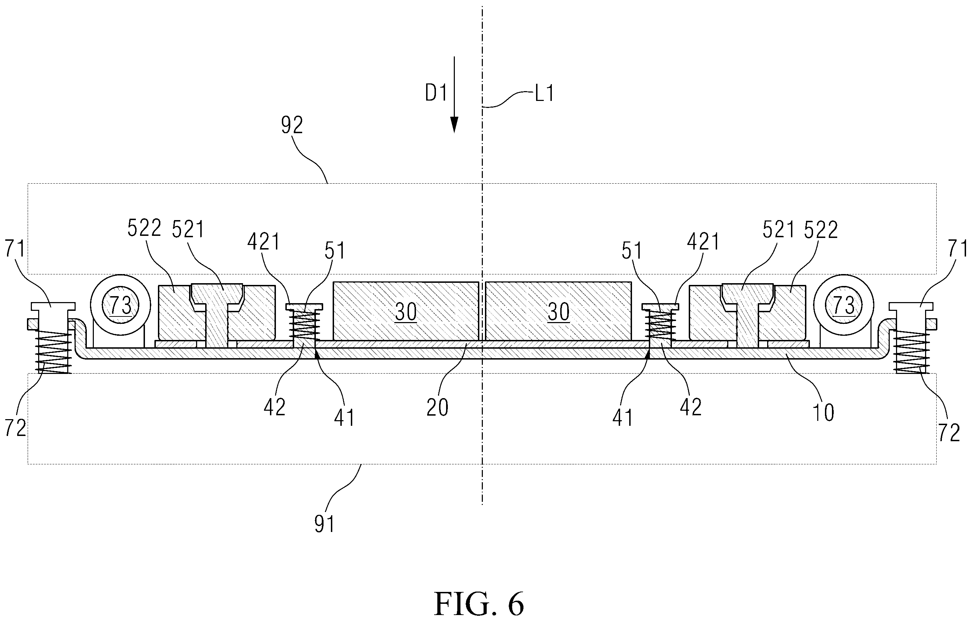

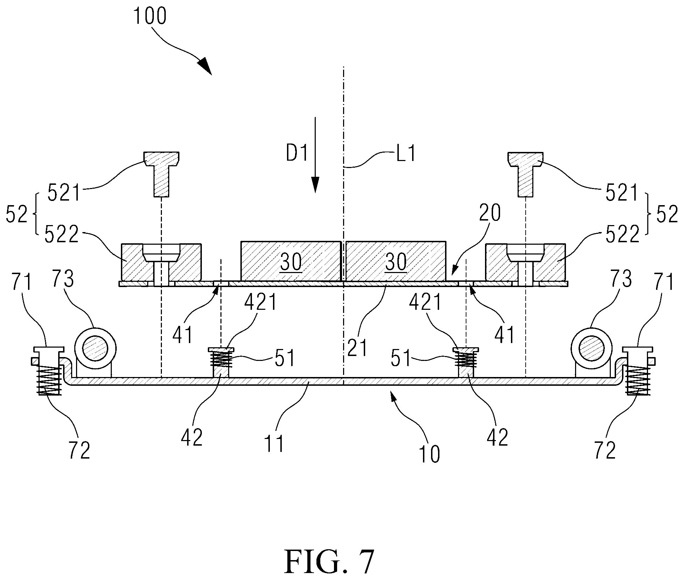

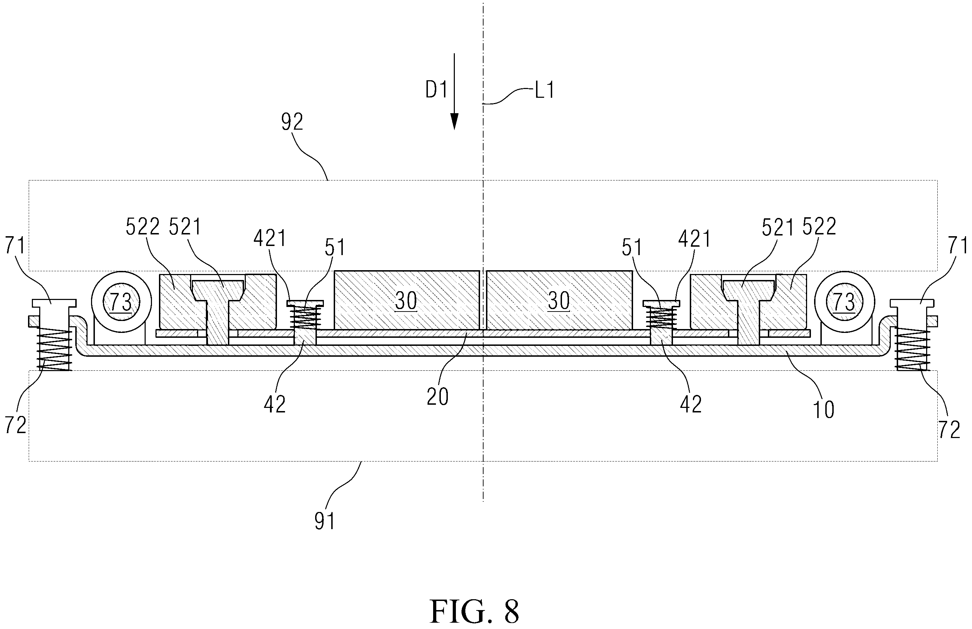

FIG. 6 is a partial sectional view of still another schematic embodiment of a braking mechanism. FIG. 7 is a schematic exploded view of the structure shown in FIG. 6. FIG. 8 is a schematic view of another state of the structure shown in FIG. 6. The parts of the braking mechanism of this schematic embodiment that are the same as or similar to the braking mechanism shown in FIG. 2 will not be repeated here, and the differences are as follows. The first magnet 30 shown in FIG. 6 is in the release position, and the first magnet 30 shown in FIG. 8 is in the braking position. As shown in FIG. 6 to FIG. 8, in this schematic embodiment, the first direction D1 is a direction in which the first magnet 30 moves from the braking position to the release position. The slide hole 41 is arranged in the second flat plate portion 21. The slide bar 42 is arranged in the first flat plate portion 11. An end of the slide bar 42 located on the second flat plate portion 21 and facing away from the first flat plate portion 11 has a boss portion 421. The first elastic member 51 is a compression spring and is sleeved on the slide bar 42. One end of the first elastic member 51 abuts against the second flat plate portion 21, and the other end abuts against the boss portion 421. In other schematic embodiments, the slide bar 42 may also be arranged to run through the first magnet 30 or the second magnet 522; and one end of the first elastic member 51 abuts against the first magnet 30 or the second magnet 522 through which the slide bar 42 runs, and the other end abuts against the boss portion 421.

In this schematic embodiment, the first magnet 30 can be driven by the elastic force of the first elastic member 51 to move from the braking position to the release position, and the magnetic force of the second magnet 522 in the driving assembly 52 applied to the iron core 521 can overcome the elastic force of the first elastic member 51, so as to drive the first magnet 30 to move from the release position to the braking position.

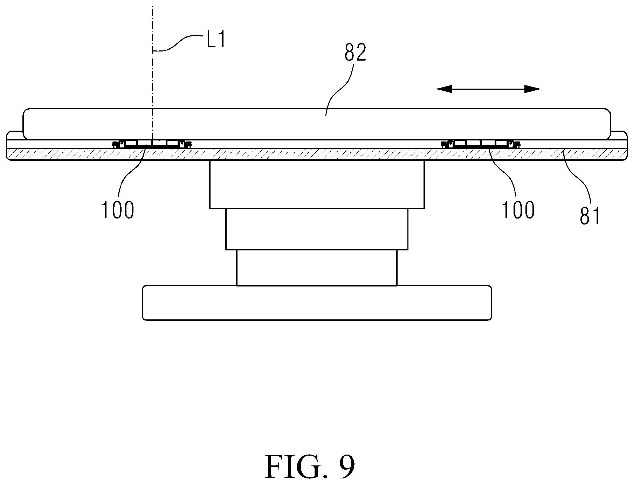

FIG. 9 is a schematic structural view of a schematic embodiment of a medical table. The medical table includes a table frame 81, a table board 82 and a plurality of the braking mechanisms 100 shown in FIG. 1, FIG. 5 or FIG. 6. The table board 82 is movably arranged on the table frame 81. The moving direction of the table board, for example, is shown as the bidirectional arrow in the figure. The moving direction is, for example, perpendicular to the first straight line L1. The first support 10 of the braking mechanism 100 is connected to the table frame 81. The table board 82 contacts the first magnet 30 in the braking position and is capable of being magnetically attracted by the magnetic force of the first magnet 30. On the basis of ensuring the braking effect, the braking mechanism of the medical table is conducive to effectively reducing the noise generated during braking.

It should be understood that although this specification is described in accordance with individual examples, not every example includes only one independent technical solution. This description manner of the specification is merely for clarity, and those skilled in the art should consider the specification as a whole. The technical solutions in the examples can also be combined as appropriate to form other embodiments that can be understood by those skilled in the art.

The series of detailed descriptions listed above are only specific descriptions of the feasible examples of the present application, and they are not intended to limit the protection scope of the present application as defined by the attached set of claims.

## Claims

1. A braking mechanism, comprising:
a first support (10), configured to connect a first object (91);
a first magnet (30), movably connected to the first support (10) along a first straight line (L1), the first magnet (30) being selected from an electromagnet and an electrically-controlled permanent magnet; and
a driving unit, capable of driving the first magnet (30) to move along the first straight line (L1) relative to the first support (10) to enable the first magnet (30) to reciprocate between a release position and a braking position, the first magnet (30) in the braking position being configured to contact and magnetically attract a second object (92) movable relative to the first object,
**characterized in that**, during braking of the braking mechanism, the driving unit firstly drives the first magnet (30) to the braking position to contact the second object (92), and then controls the first magnet (30) to generate a magnetic field to attract the second object (92).

2. The braking mechanism according to claim 1, wherein the driving unit comprises:
a first elastic member (51), capable of applying an elastic force to drive the first magnet (30) to move between the release position and the braking position along a first direction (D1) parallel to the first straight line (L1) relative to the first support (10); and
a driving assembly (52), comprising an iron core (521) and a second magnet (522), the iron core (521) movably running through the second magnet (522) along the first straight line (L1), the second magnet (522) being selected from an electromagnet and an electrically-controlled permanent magnet, a magnetic field generated by the second magnet (522) being capable of driving the iron core (521) to move along the first straight line (L1) relative to the second magnet (522), one of the iron core (521) and the second magnet (522) being fixedly arranged relative to the first support (10), and the other one of the iron core (521) and the second magnet (522) being fixedly arranged relative to the first magnet (30) to drive the iron core (521) to move by the second magnet (522) to drive the first magnet (30) to move between the release position and the braking position along a direction opposite to the first direction (D1).

3. The braking mechanism according to claim 2, wherein the braking mechanism further comprises a second support (20), the second support (20) is movably connected to the first support (10) along the first straight line (L1), the first magnet (30) and the second magnet (522) are fixedly arranged on the second support (20), and the iron core (521) are fixedly arranged on the first support (10).

4. The braking mechanism according to claim 3, wherein the first support (10) has a first flat plate portion (11), the second support (20) has a second flat plate portion (21), the first flat plate portion (11) and the second flat plate portion (21) are both arranged perpendicular to the first direction (D1) and overlapped with each other along the first direction (D1), and the first magnet (30) and the second magnet (522) are arranged on a side of the second flat plate portion (21) facing away from the first flat plate portion (11).

5. The braking mechanism according to claim 4, wherein one of the first flat plate portion (11) and the second flat plate portion (21) is provided with a slide hole (41) penetrating along the first direction (D1), and the other one of the first flat plate portion (11) and the second flat plate portion (21) is provided with a slide bar (42), the slide bar (42) slidably running through the slide hole (41) along the first direction (D1).

6. The braking mechanism according to claim 5, wherein the first direction (D1) is a direction in which the first magnet (30) moves from the braking position to the release position, the second flat plate portion (21) has the slide hole (41), the first support (10) has the slide bar (42), an end of the slide bar (42) located on the second flat plate portion (21) and facing away from the first flat plate portion (11) has a boss portion (421), the first elastic member (51) is a compression spring and is sleeved on the slide bar (42), one end of the first elastic member (51) abuts against the second flat plate portion (21), the first magnet (30) or the second magnet (522), and the other end abuts against the boss portion (421).

7. The braking mechanism according to claim 4, wherein the first direction (D1) is a direction in which the first magnet (30) moves from the release position to the braking position, the second flat plate portion (21) has an avoiding hole (211), the first magnet (30) has a first accommodating cavity (31) opposite to the avoiding hole (211) along the first direction (D1), the first elastic member (51) runs through the avoiding hole (211), one end of the first elastic member (51) abuts against the first flat plate portion (11), and the other end extends into the first accommodating cavity (31) and abuts against the first magnet (30).

8. The braking mechanism according to claim 1, wherein the braking mechanism is provided with a plurality of the first magnets (30), and at least a part of the plurality of first magnets (30) are electrically-controlled permanent magnets.

9. The braking mechanism according to claim 1, wherein the braking mechanism further comprises:
a connecting member (71), movably connected to the first support (10) along the first straight line (L1), and the first support (10) being connected to the first object through the connecting member (71);
a second elastic member (72), capable of applying an elastic force to drive the first support (10) to move relative to the connecting member (71) in a direction the same as the direction in which the first magnet (30) moves from the release position to the braking position; and
an abutting member (73), connected to the first support (10) to abut against the second object under the action of the elastic force of the second elastic member (72).

10. The braking mechanism according to claim 9, wherein the abutting member (73) is a roller wheel, and the roller wheel is rotatably connected to the first support (10) with a rotation axis perpendicular to the first straight line (L1).

11. The braking mechanism according to claim 9, wherein the connecting member (71) is a pin and runs through the first support (10), and the second elastic member (72) is a compression spring and is sleeved on the connecting member (71).

12. The braking mechanism according to claim 11, wherein the braking mechanism is provided with a plurality of the connecting members (71), and the first support (10) comprises:
a first flat plate portion (11), being in a flat plate shape perpendicular to the first straight line (L1); and
a plurality of connecting portions (12), each of the connecting portions (12) being in a flat plate shape parallel to the first flat plate portion (11), each of the connecting members (71) running through one of the connecting portions (12), and the first magnet (30), the abutting member (73) and the connecting portions (12) being located on a same side of the first flat plate portion (11).

13. A medical table, comprising:
a table frame (81);
a table board (82), movably arranged on the table frame (81); and
a braking mechanism according to any one of claims 1 to 12, the first support (10) being connected to one of the table frame (81) and the table board (82), and the other one of the table frame (81) and the table board (82) contacting the first magnet (30) in the braking position and being capable of being magnetically attracted by the magnetic force of the first magnet (30).

## Patentansprüche

1. Bremsmechanismus, umfassend:
eine erste Stütze (10), die dazu ausgelegt ist, sich mit einem ersten Objekt (91) zu verbinden;
einen ersten Magneten (30), der beweglich mit der ersten Stütze (10) entlang einer geraden Linie (L1) verbunden ist, wobei der erste Magnet (30) aus einem Elektromagneten und einem elektrisch gesteuerten Dauermagneten ausgewählt ist; und
eine Antriebseinheit, die in der Lage ist, den ersten Magneten (30) dahingehend anzutreiben, sich entlang der geraden Linie (L1) bezogen auf die erste Stütze (10) zu bewegen, um zu ermöglichen, dass sich der erste Magnet (30) zwischen einer Freigabeposition und einer Bremsposition hin- und her bewegt, wobei der erste Magnet (30) in der Bremsposition dazu ausgelegt ist, ein zweites Objekt (92), das bezogen auf das erste Objekt beweglich ist, zu kontaktieren und magnetisch anzuziehen,
**dadurch gekennzeichnet, dass**, beim Bremsen des Bremsmechanismus, die Antriebseinheit zunächst den ersten Magneten (30) in die Bremsposition treibt, um das zweite Objekt (92) zu kontaktieren, und dann den ersten Magneten (30) steuert, um ein Magnetfeld zu erzeugen, um das zweite Objekt (92) anzuziehen.

2. Bremsmechanismus nach Anspruch 1, wobei die Antriebseinheit umfasst:
ein erstes elastisches Element (51), das in der Lage ist, eine elastische Kraft aufzubringen, um den ersten Magneten (30) dahingehend anzutreiben, sich zwischen der Freigabeposition und der Bremsposition entlang einer ersten Richtung (D1) parallel zu der ersten geraden Linie (L1) bezogen auf die erste Stütze (10) zu bewegen; und
eine Antriebsanordnung (52), umfassend einen Eisenkern (521) und einen zweiten Magneten (522), wobei der Eisenkern (521) beweglich entlang der ersten geraden Linie (L1) durch den zweiten Magneten (522) läuft, wobei der zweite Magnet (522) aus einem Elektromagneten und einem elektrisch gesteuerten Dauermagneten ausgewählt ist, wobei ein von dem zweiten Magneten (522) erzeugtes Magnetfeld in der Lage ist, den Eisenkern (521) dahingehend anzutreiben, sich entlang der ersten geraden Linie (L1) bezogen auf den zweiten Magneten (522) zu bewegen, wobei einer von dem Eisenkern (521) und dem zweiten Magneten (522) bezogen auf die erste Stütze (10) fest angeordnet ist, und wobei der andere von dem Eisenkern (521) und dem zweiten Magneten (522) bezogen auf den ersten Magneten (30) fest angeordnet ist, um den Eisenkern (521) dahingehend anzutreiben, sich durch den zweiten Magneten (522) zu bewegen, um den ersten Magneten (30) zwischen der Freigabeposition und der Bremsposition entlang einer Richtung entgegen der ersten Richtung (D1) zu bewegen.

3. Bremsmechanismus nach Anspruch 2, wobei der Bremsmechanismus ferner eine zweite Stütze (20) umfasst, die zweite Stütze (20) beweglich mit der ersten Stütze (10) entlang der ersten geraden Linie (L1) verbunden ist, der erste Magnet (30) und der zweite Magnet (522) fest an der zweiten Stütze (20) angeordnet sind und der Eisenkern (521) fest an der ersten Stütze (10) angeordnet ist.

4. Bremsmechanismus nach Anspruch 3, wobei die erste Stütze (10) einen ersten flachen Plattenabschnitt (11) hat, die zweite Stütze (20) einen zweiten flachen Plattenabschnitt (21) hat, der erste flache Plattenabschnitt (11) und der zweite flache Plattenabschnitt (21) beide senkrecht zu der ersten Richtung (D1) und einander überlappend entlang der ersten Richtung (D1) angeordnet sind und der erste Magnet (30) und der zweite Magnet (522) an einer Seite des zweiten flachen Plattenabschnitts (21) angeordnet sind, der von dem ersten flachen Plattenabschnitt (11) abgewandt ist.

5. Bremsmechanismus nach Anspruch 4, wobei einer von dem ersten flachen Plattenabschnitt (11) und dem zweiten flachen Plattenabschnitt (21) mit einem Gleitloch (41) bereitgestellt ist, das entlang der ersten Richtung (D1) durchgeht, und der andere von dem ersten flachen Plattenabschnitt (11) und dem zweiten flachen Plattenabschnitt (21) mit einer Gleitstange (42) bereitgestellt ist, wobei die Gleitstange (42) verschiebbar durch das Gleitloch (41) entlang der ersten Richtung (D1) läuft.

6. Bremsmechanismus nach Anspruch 5, wobei die erste Richtung (D1) eine Richtung ist, in der sich der erste Magnet (30) aus der Bremsposition in die Freigabeposition bewegt, der zweite flache Plattenabschnitt (21) das Gleitloch (41) hat, die erste Stütze (10) die Gleitstange (42) hat, ein Ende der Gleitstange (42), die sich an dem zweiten flachen Plattenabschnitt (21) befindet und von dem ersten flachen Plattenabschnitt (11) abgewandt ist, einen Vorsprungsabschnitt (421) hat, das erste elastische Element (51) eine Druckfeder ist und zur Ummantelung auf die Gleitstange (42) geschoben ist, ein Ende des ersten elastischen Elements (51) an dem zweiten flachen Plattenabschnitt (21) anliegt, der erste Magnet (30) oder der zweite Magnet (522) und das andere Ende an dem Vorsprungsabschnitt (421) anliegen.

7. Bremsmechanismus nach Anspruch 4, wobei die erste Richtung (D1) eine Richtung ist, in der sich der erste Magnet (30) aus der Freigabeposition in die Bremsposition bewegt, der zweite flache Plattenabschnitt (21) ein Vermeidungsloch (211) hat, der erste Magnet (30) einen ersten Aufnahmehohlraum (31) gegenüber dem Vermeidungsloch (211) entlang der ersten Richtung (D1) hat, das erste elastische Element (51) durch das Vermeidungsloch (211) läuft, ein Ende des ersten elastischen Elements (51) an dem ersten flachen Plattenabschnitt (11) anliegt und sich das andere Ende in den ersten Aufnahmehohlraum (31) erstreckt und an dem ersten Magneten (30) anliegt.

8. Bremsmechanismus nach Anspruch 1, wobei der Bremsmechanismus mit einer Vielzahl der ersten Magnete (30) bereitgestellt ist und mindestens ein Teil der Vielzahl der ersten Magnete (30) elektrisch gesteuerte Dauermagnete sind.

9. Bremsmechanismus nach Anspruch 1, wobei der Bremsmechanismus ferner umfasst:
ein Verbindungselement (71), das beweglich mit der ersten Stütze (10) entlang der ersten geraden Linie (L1) verbunden ist und wobei die erste Stütze (10) durch das Verbindungselement (71) mit dem ersten Objekt verbunden ist;
ein zweites elastisches Element (72), das in der Lage ist, eine elastische Kraft aufzubringen, um die erste Stütze (10) dahingehend anzutreiben, sich bezogen auf das Verbindungselement (71) in eine Richtung zu bewegen, die dieselbe ist wie die Richtung, in die sich das erste Magnet (30) aus der Freigabeposition in die Bremsposition bewegt; und
ein Anlageelement (73), das mit der ersten Stütze (10) verbunden ist, um unter der Einwirkung der elastischen Kraft des zweiten elastischen Elements (72) an dem zweiten Objekt anzuliegen.

10. Bremsmechanismus nach Anspruch 9, wobei das Anlageelement (73) ein Rollenrad ist und das Rollenrad drehbar mit der ersten Stütze (10) verbunden ist, wobei eine Drehachse senkrecht zu der ersten geraden Linie (L1) ist.

11. Bremsmechanismus nach Anspruch 9, wobei das Verbindungselement (71) ein Stift ist und durch die erste Stütze (10) läuft und das zweite elastische Element (72) eine Druckfeder ist und zur Ummantelung auf das Verbindungselement (71) geschoben ist.

12. Bremsmechanismus nach Anspruch 11, wobei der Bremsmechanismus mit einer Vielzahl von Verbindungselementen (71) bereitgestellt ist und die erste Stütze (10) umfasst:
einen ersten flachen Plattenabschnitt (11), der eine flache Plattenform senkrecht zu der ersten geraden Linie (L1) hat; und
eine Vielzahl von Verbindungsabschnitten (12), wobei jeder der Verbindungsabschnitte (12) eine flache Plattenform parallel zu dem ersten flachen Plattenabschnitt (11) hat, wobei jedes der Verbindungselemente (71) durch einen der Verbindungsabschnitte (12) läuft und wobei sich der erste Magnet (30), das Anlageelement (73) und die Verbindungsabschnitte (12) auf derselben Seite des ersten flachen Plattenabschnitts (11) befinden.

13. Medizinischer Tisch, umfassend:
ein Tischgestell (81),
eine Tischplatte (82), die beweglich auf dem Tischgestell (81) angeordnet ist; und
einen Bremsmechanismus nach einem der Ansprüche 1 bis 12, wobei die erste Stütze (10) mit einem von dem Tischgestell (81) und der Tischplatte (82) verbunden ist und der andere von dem Tischgestell (81) und der Tischplatte (82) den ersten Magneten (30) in der Bremsposition kontaktiert und in der Lage ist, von der Magnetkraft des ersten Magneten (30) magnetisch angezogen zu werden.

## Revendications

1. Mécanisme de freinage, comprenant :
un premier support (10), conçu pour relier un premier objet (91) ;
un premier aimant (30), relié de manière mobile au premier support (10) le long d'une première ligne droite (L1), le premier aimant (30) étant sélectionné parmi un électroaimant et un aimant permanent commandé électriquement ; et
une unité d'entraînement, susceptible d'entraîner le premier aimant (30) en déplacement le long de la première ligne droite (L1) par rapport au premier support (10) afin de permettre au premier aimant (30) d'aller et venir entre une position de libération et une position de freinage, le premier aimant (30) dans la position de freinage étant conçu pour entrer en contact avec un second objet (92) mobile par rapport au premier objet, et attirer magnétiquement celui-ci,
**caractérisé en ce que**, pendant le freinage du mécanisme de freinage, l'unité d'entraînement entraîne d'abord le premier aimant (30) vers la position de freinage pour entrer en contact avec le second objet (92), puis commande le premier aimant (30) pour générer un champ magnétique pour attirer le second objet (92).

2. Mécanisme de freinage selon la revendication 1, dans lequel l'unité d'entraînement comprend :
un premier élément élastique (51), susceptible d'appliquer une force élastique pour entraîner le premier aimant (30) en déplacement entre la position de libération et la position de freinage le long d'une première direction (D1) parallèle à la première ligne droite (L1) par rapport au premier support (10) ; et
un ensemble d'entraînement (52), comprenant un noyau de fer (521) et un second aimant (522), le noyau de fer (521) se déplaçant à travers le second aimant (522) le long de la première ligne droite (L1), le second aimant (522) étant sélectionné parmi un électroaimant et un aimant permanent commandé électriquement, un champ magnétique généré par le second aimant (522) étant susceptible d'entraîner le noyau de fer (521) en déplacement le long de la première ligne droite (L1) par rapport au second aimant (522), l'un parmi le noyau de fer (521) et le second aimant (522) étant disposé à demeure par rapport au premier support (10), et l'autre parmi le noyau de fer (521) et le second aimant (522) étant disposé à demeure par rapport au premier aimant (30) pour entraîner le noyau de fer (521) en déplacement par le second aimant (522) pour entraîner le premier aimant (30) en déplacement entre la position de libération et la position de freinage le long d'une direction opposée à la première direction (D1).

3. Mécanisme de freinage selon la revendication 2, dans lequel le mécanisme de freinage comprend en outre un second support (20), le second support (20) est relié de manière mobile au premier support (10) le long de la première ligne droite (L1), le premier aimant (30) et le second aimant (522) sont disposés à demeure sur le second support (20), et le noyau de fer (521) est disposé à demeure sur le premier support (10).

4. Mécanisme de freinage selon la revendication 3, dans lequel le premier support (10) a une première partie plaque plate (11), le second support (20) a une seconde partie plaque plate (21), la première partie plaque plate (11) et la seconde partie plaque plate (21) sont toutes deux agencées perpendiculairement à la première direction (D1) et se chevauchent l'une l'autre le long de la première direction (D1), et le premier aimant (30) et le second aimant (522) sont disposés sur un côté de la seconde partie plaque plate (21) à l'opposé de la première partie plaque plate (11).

5. Mécanisme de freinage selon la revendication 4, dans lequel l'une parmi la première partie plaque plate (11) et la seconde partie plaque plate (21) est pourvue d'un trou de glissement (41) pénétrant le long de la première direction (D1), et l'autre parmi la première partie plaque plate (11) et la seconde partie plaque plate (21) est pourvue d'une barre de glissement (42), la barre de glissement (42) glissant à travers le trou de glissement (41) le long de la première direction (D1).

6. Mécanisme de freinage selon la revendication 5, dans lequel la première direction (D1) est une direction dans laquelle le premier aimant (30) se déplace de la position de freinage à la position de libération, la seconde partie plaque plate (21) comporte le trou de glissement (41), le premier support (10) comporte la barre de glissement (42), une extrémité de la barre de glissement (42) située sur la seconde partie plaque plate (21) et tournée à l'opposé de la première partie plaque plate (11) comporte une partie bossage (421), le premier élément élastique (51) est un ressort de compression et est manchonné sur la barre de glissement (42), une extrémité du premier élément élastique (51) bute contre la seconde partie plaque plate (21), le premier aimant (30) ou le second aimant (522), et l'autre extrémité bute contre la partie bossage (421).

7. Mécanisme de freinage selon la revendication 4, dans lequel la première direction (D1) est une direction dans laquelle le premier aimant (30) se déplace de la position de libération à la position de freinage, la seconde partie plaque plate (21) a un trou d'évitement (211), le premier aimant (30) a une première cavité de réception (31) en regard du trou d'évitement (211) le long de la première direction (D1), le premier élément élastique (51) passe à travers le trou d'évitement (211), une extrémité du premier élément élastique (51) bute contre la première partie plaque plate (11), et l'autre extrémité s'étend dans la première cavité de réception (31) et bute contre le premier aimant (30).

8. Mécanisme de freinage selon la revendication 1, dans lequel le mécanisme de freinage est pourvu d'une pluralité des premiers aimants (30), et au moins une partie de la pluralité de premiers aimants (30) est des aimants permanents commandés électriquement.

9. Mécanisme de freinage selon la revendication 1, le mécanisme de freinage comprenant en outre :
un élément de liaison (71), relié de manière mobile au premier support (10) le long de la première ligne droite (L1), et le premier support (10) étant relié au premier objet par l'intermédiaire de l'élément de liaison (71) ;
un second élément élastique (72), susceptible d'appliquer une force élastique pour entraîner le premier support (10) en déplacement par rapport à l'élément de liaison (71) dans une direction identique à la direction dans laquelle le premier aimant (30) se déplace de la position de libération à la position de freinage ; et
un élément de butée (73), relié au premier support (10), pour buter contre le second objet sous l'action de la force élastique du second élément élastique (72).

10. Mécanisme de freinage selon la revendication 9, dans lequel l'élément de butée (73) est une roue à rouleaux, et la roue à rouleaux est reliée de manière rotative au premier support (10) avec un axe de rotation perpendiculaire à la première ligne droite (L1).

11. Mécanisme de freinage selon la revendication 9, dans lequel l'élément de liaison (71) est une broche et traverse le premier support (10), et le second élément élastique (72) est un ressort de compression et est manchonné sur l'élément de liaison (71).

12. Mécanisme de freinage selon la revendication 11, dans lequel le mécanisme de freinage est pourvu d'une pluralité des éléments de liaison (71), et le premier support (10) comprend :
une première partie plaque plane (11), ayant une forme de plaque plate perpendiculaire à la première ligne droite (L1) ; et
une pluralité de parties de liaison (12), chacune des parties de liaison (12) étant en forme de plaque plate parallèle à la première partie plaque plate (11), chacun des éléments de liaison (71) passant à travers l'une des parties de liaison (12), et le premier aimant (30), l'élément de butée (73) et les parties de liaison (12) étant situés sur un même côté de la première partie plaque plate (11).

13. Table médicale, comprenant :
un cadre de table (81) ;
un plateau de table (82), disposé de manière mobile sur le cadre de table (81) ; et
un mécanisme de freinage selon l'une quelconque des revendications 1 à 12, le premier support (10) étant relié à l'un parmi le cadre de table (81) et le plateau de table (82), et l'autre parmi le cadre de table (81) et le plateau de table (82) étant en contact avec le premier aimant (30) dans la position de freinage et pouvant être attiré magnétiquement par la force magnétique du premier aimant (30).
